# EUROPEAN PATENT APPLICATION

(11) **EP 4 582 549 A1**
(43) Date of publication of application: **09.07.2025**
(21) Application number: 23860545.5
(22) Date of filing: 01.09.2023
(51) Int. Cl.: C12P 7/24, A23L 5/00, C11B 9/00, C12N 9/02, A23L 27/20, C07C 47/228

(54) **PHENYLACETALDEHYDE PRODUCTION METHOD AND USE APPLICATION THEREOF**

(30) Priority: 02.09.2022 JP 2022139959; 26.05.2023 JP 2023087203
(71) Applicant: Amano Enzyme Inc., Nagoya-shi Aichi 460-8630 (JP)
(72) Inventor: SATO Yukihide, Kakamigahara-shi, Gifu 509-0109 (JP)
(74) Representative: Witthoff Jaekel Steinecke Patentanwälte PartG mbB
(86) International application number: PCT/JP2023/032116
(87) International publication number: WO 2024/048785

(57) **Abstract**

It is an object of the present invention to establish a method for producing phenylacetaldehyde that is safe, environmentally friendly, and efficient. The present invention relates to a method for producing phenylacetaldehyde, including allowing a mediator to act on phenylalanine or an analogue thereof, in which the mediator is a compound containing a ring structure having at least one hydroxyl group. **In** addition, the present invention relates to a phenylacetaldehyde-generating enzyme agent, an enzyme agent for improving the flavor of food products and beverages, and a method for improving the flavor of food products and beverages.

## Description

### Technical Field

The present invention relates to a method for producing phenylacetaldehyde and a phenylacetaldehyde-generating enzyme agent.

### Background Art

Phenylacetaldehyde is used as a raw material for flavorings, medicaments, pesticides, artificial sweeteners and the like, and is also a substance that is important as an intermediate for amino acids. Since phenylacetaldehyde exhibits a sweet scent, a rose-like scent, and a fresh scent, it is mainly used as a raw material for blending flavors in flavorings or food products.

Moreover, phenylacetaldehyde is known to be one of components that increase when a food product is heated, and is also known to be a component that gives a rich taste. For example, Patent Document 1 discloses that heat-treating cruciferous vegetables increases phenylacetaldehyde, thereby contributing to the rich taste.

Various methods for generating phenylacetaldehyde, other than heat-treating cruciferous vegetables, etc., have been studied. For example, Patent Document 2 discloses a method for generating aromatic acetaldehyde by organic synthesis. Patent Document 3 discloses a method for generating phenylacetaldehyde by allowing tyrosinase and phenylalanine to act on each other, and Patent Document 4 discloses a method for generating phenylacetaldehyde from phenylalanine by allowing rose-derived pyridoxal phosphate (PLP)-dependent aromatic decarboxylase to react with phenylalanine in the presence of PLP.

### Prior Art Documents

### Patent Documents

Patent Document 1: International Publication No. WO2018/207818
Patent Document 2: JP Patent Publication No. 5-229981 A (1993)
Patent Document 3: JP Patent Publication No. 2003-310289 A
Patent Document 4: JP Patent Publication No. 2007-189943 A

### Summary of Invention

### Objects to be Solved by the Invention

Since phenylacetaldehyde exhibits a floral scent, its addition into a food product is expected to mask off-flavors and to improve the flavor. However, there is still room for improvement in improving the flavor of food products. Then, it has been desired to establish a novel method for producing phenylacetaldehyde that is safe, environmentally friendly, and efficient.

Thus, it is an object of the present invention to establish a method for producing phenylacetaldehyde that is safe, environmentally friendly, and efficient.

### Means for Solving the Objects

As a result of intensive studies conducted directed towards achieving the aforementioned object, the present inventors have found that phenylacetaldehyde is generated and a floral scent is exhibited by establishing a step of allowing a mediator having a predetermined structure to act on phenylalanine or an analogue thereof, thereby completing the present invention.

Specifically, the present invention has the following configurations.
[1] A method for producing phenylacetaldehyde, including
   allowing a mediator to act on phenylalanine or an analogue thereof, in which
   the mediator is a compound containing a ring structure having at least one hydroxyl group.
[2] The method for producing phenylacetaldehyde according to [1], in which the allowing the mediator to act on phenylalanine or an analogue thereof is carried out in the presence of multicopper oxidase.
[3] The method for producing phenylacetaldehyde according to [1] or [2], in which the mediator is a compound containing a ring structure having at least one hydroxyl group and an alkoxy group, or a compound containing a ring structure having two or more hydroxyl groups.
[4] The method for producing phenylacetaldehyde according to any one of [1] to [3], in which the ring structure is a benzene ring or a dihydrofuran ring.
[5] The method for producing phenylacetaldehyde according to any one of [1] to [4], in which the mediator is at least one type selected from the group consisting of DL-catechin, tannic acid, chlorogenic acid, caffeic acid, curcumin, hesperidin, L-DOPA, pyrogallol, vanillin, eugenol, gallic acid monohydrate, ferulic acid, isoeugenol, α-glucosylrutin and rutin.
[6] The method for producing phenylacetaldehyde according to any one of [1] to [5], in which the mediator is a compound contained in at least one type selected from the group consisting of an apple extract, pectin, a green coffee bean extract and a tea extract.
[7] The method for producing phenylacetaldehyde according to any one of [1] to [6], in which the mediator is a polyphenol.
[8] The method for producing phenylacetaldehyde according to any one of [1] to [7], in which the mediator is at least one type selected from the group consisting of α-glucosylrutin, chlorogenic acid, caffeic acid, tannic acid and rutin.
[9] The method for producing phenylacetaldehyde according to any one of [2] to [8], in which the multicopper oxidase is at least one type selected from phenol oxidase and bilirubin oxidase.
[10] The method for producing phenylacetaldehyde according to any one of [2] to [9], in which the multicopper oxidase is laccase.
[11] The method for producing phenylacetaldehyde a according to any one of [1] to [10], in which the reaction temperature in the allowing the mediator to act on phenylalanine or an analogue thereof is 60°C or lower.
[12] A phenylacetaldehyde-generating enzyme agent containing a mediator and multicopper oxidase, in which
   the mediator is a compound containing a ring structure having at least one hydroxyl group.

Also, the present invention has the following configurations.
[A] Phenylacetaldehyde produced by the method for producing phenylacetaldehyde according to any one of the above [1] to [11].
[B] A method for improving the flavor of food products and beverages, including allowing a mediator containing a ring structure having at least one hydroxyl group and multicopper oxidase to act on phenylalanine or an analogue thereof.
[C] An enzyme agent, which contains a mediator containing a ring structure having at least one hydroxyl group and multicopper oxidase, the enzyme agent for use in improving the flavor of food products and beverages.
[D] Use of an enzyme agent, which contains a mediator containing a ring structure having at least one hydroxyl group and multicopper oxidase, for improving the flavor of food products and beverages.
[E] An enzyme agent for improving the flavor of food products and beverages, which contains a mediator and multicopper oxidase, in which
   the mediator is a compound containing a ring structure having at least one hydroxyl group.
[F] A method for improving the flavor of food products and beverages, including a step of allowing a mediator and multicopper oxidase to act on phenylalanine or an analogue thereof, in which
   the mediator is a compound containing a ring structure having at least one hydroxyl group.

### Advantageous Effects of Invention

According to the present invention, there can be provided a method for producing phenylacetaldehyde that is safe, environmentally friendly, and efficient. In addition, according to the present invention, there can also be provided a phenylacetaldehyde-generating enzyme agent, an enzyme agent for improving the flavor of food products and beverages, and a method for improving the flavor of food products and beverages.

### Embodiments of Carrying out the Invention

Hereafter, the present invention will be described in detail. The configuration requirements described below may be described based on representative embodiments or specific examples, but the present invention is not limited to such embodiments. In the present description, a numerical value range expressed using the preposition "to" means a range that includes the numerical values described before and after the preposition "to" as a lower limit value and a upper limit value, respectively.

### (Method for producing phenylacetaldehyde)

The present invention relates to a method for producing phenylacetaldehyde, including a step of allowing a mediator to act on phenylalanine or an analogue thereof. The mediator is a compound containing a ring structure having at least one hydroxyl group. Since the present invention has the above-described configuration, phenylacetaldehyde can be produced in consideration of safety and environment, with good efficiency. Since the phenylacetaldehyde obtained by the production method of the present invention has high safety and can be added to food products, it becomes possible to mask off-flavors in food products and to improve the flavor of food products.

In the present invention, the step of allowing a mediator to act on phenylalanine or an analogue thereof is preferably a step that is carried out in the presence of multicopper oxidase. That is to say, the present invention preferably relates to a method for producing phenylacetaldehyde, including a step of allowing a mediator to act on phenylalanine or an analogue thereof in the presence of multicopper oxidase. By carrying out the step of allowing a mediator to act on phenylalanine or an analogue thereof in the presence of multicopper oxidase, the amount of phenylacetaldehyde generated can be increased, and phenylacetaldehyde can be more efficiently produced.

The step of allowing a mediator to act on phenylalanine or an analogue thereof in the present invention may also be a step of allowing the mediator having the above-described predetermined structure to act on phenylalanine or an analogue thereof, in food products and beverages containing phenylalanine, or in food products and beverages to which phenylalanine has been added. Otherwise, the above-described step may also be a step of allowing the mediator having the above-described predetermined structure and multicopper oxidase to act on phenylalanine or an analogue thereof, in food products and beverages containing phenylalanine, or in food products and beverages to which phenylalanine has been added. In general, phenylacetaldehyde is said to be instable. However, by allowing the mediator to act on food products and beverages containing phenylalanine or food products and beverages to which phenylalanine has been added, phenylacetaldehyde does not need to be isolated, and it also becomes possible to directly obtain food products and beverages containing phenylacetaldehyde.

It is to be notes that the step of producing phenylacetaldehyde of the present invention may be included a method for producing food products and beverages containing phenylacetaldehyde. In this case, the method for producing food products and beverages containing phenylacetaldehyde may include a step of allowing the mediator having the above-described predetermined structure on phenylalanine or an analogue thereof, in materials for food products and beverages containing phenylalanine, or in materials for food products and beverages to which phenylalanine has been added. Moreover, the above-described step may also be a step of allowing the mediator having the above-described predetermined structure and multicopper oxidase to act on phenylalanine or an analogue thereof, in materials for food products and beverages containing phenylalanine, or in materials for food products and beverages to which phenylalanine has been added.

Phenylalanine is one type of amino acid, and is a component present in many types of food products. In the method for producing phenylacetaldehyde of the present invention, such phenylalanine is used as a raw material, and phenylacetaldehyde is produced by allowing a mediator and multicopper oxidase to act on phenylalanine or an analogue thereof. Since a food product-derived compound or a highly safe compound can be used as such a mediator or multicopper oxidase, the resulting phenylacetaldehyde is highly safe and can be used in pharmaceutical products, or in food products and beverages. In addition, the method for producing phenylacetaldehyde of the present invention does not involve organic synthesis, and the reaction can be carried out in an aqueous solvent, so that the burden on the environment can be reduced.

Moreover, in the present invention, benzaldehyde can be produced in addition to phenylacetaldehyde by allowing a mediator and multicopper oxidase to act on phenylalanine or an analogue thereof. Since benzaldehyde has a sweet, fruity scent, it is preferably used in seasonings and various processed food products. Furthermore, benzaldehyde is also known to have antibacterial activity against *Staphylococcus aureus* and other bacteria, and thus, it useful as an antibacterial ingredient.

### < Phenylalanine >

In the method for producing phenylacetaldehyde of the present invention, phenylalanine or an analogue thereof is used as a reaction substrate (oxidation substrate). As a phenylalanine analogue, it is preferable that at least one hydrogen atom in the side chain of phenylalanine is substituted with a substituent; and it is more preferable that the hydrogen atom in the benzene ring in the side chain of phenylalanine is substituted. Examples of the substituent may include: halogen atoms such as fluorine, chlorine, bromine and iodine; ethynyl groups; cyano groups; and azide groups.

The phenylalanine used in the method for producing phenylacetaldehyde of the present invention is preferably L-phenylalanine (LPA), a naturally occurring compound, but may also be D-phenylalanine (DPA), an artificially produced compound. In addition, a food product containing L-phenylalanine, to which D-phenylalanine has been added, may be used, or a food product containing neither L-phenylalanine nor D-phenylalanine, to which L-phenylalanine and D-phenylalanine have been added, may also be used.

The concentration of the phenylalanine used in the method for producing phenylacetaldehyde of the present invention is preferably adjusted, as appropriate, depending on the type and concentration of the mediator or multicopper oxidase to be acted on the phenylalanine or an analogue thereof. For example, the initial concentration of phenylalanine in the step of allowing a mediator to act on phenylalanine or an analogue thereof is preferably 0.01 mM or more, more preferably 0.1 mM or more, even more preferably 1 mM or more, further preferably 5 mM or more, and particularly preferably 10 mM or more. On the other hand, the initial concentration of phenylalanine is preferably 1000 mM or less, more preferably 900 mM or less, even more preferably 800 mM or less, further preferably 700 mM or less, and particularly preferably 500 mM or less. The initial concentration of phenylalanine may also be 200 mM or less, 150 mM or less, 100 mM or less, 50 mM or less, 30 mM or less, or 20 mM or less. Phenylacetaldehyde can be efficiently produced by setting the initial concentration of phenylalanine to be within the above-described range.

### < Mediator >

The method for producing phenylacetaldehyde of the present invention includes a step of allowing a mediator to act on phenylalanine or an analogue thereof. Herein, the mediator is a compound containing a ring structure having at least one hydroxyl group. Besides, in the present description, the mediator may be a reducing substrate. In addition, the mediator is preferably a component or a compound that plays a role for promoting or mediating generation of phenylacetaldehyde from phenylalanine. In the step of producing phenylacetaldehyde of the present invention, it is considered that the hydroxyl group possessed by the ring structure of the mediator is oxidized, and the oxidized compound acts on phenylalanine or an analogue thereof to cause Strecker degradation, thereby generating phenylacetaldehyde.

The mediator may be one that contains one ring structure having at least one hydroxyl group in one molecule thereof, or may also be one that contains two or more of the ring structures.

The mediator is preferably a compound that contains a ring structure having at least one hydroxyl group and an alkoxy group, or a compound that contains a ring structure having two or more hydroxyl groups. For example, the mediator is preferably a compound having the following structure:

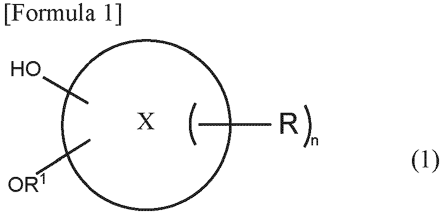

In the formula (1), X represents a ring structure, R represents a substituent, n represents a natural number including 0, with the upper limit being a substitutable number, and R¹ represents a hydrogen atom or an alkyl group.

The ring structure represented by X is preferably a heterocyclic ring or a cyclic hydrocarbon. The ring structure represented by X is preferably a 3-membered ring to a 10-membered ring, more preferably a 4-membered ring to a 8-membered ring, and further preferably a 5-membered ring or a 6-membered ring. When the ring structure represented by X is a heterocyclic ring, examples of the heteroatom constituting the heterocyclic ring may include an oxygen atom, a nitrogen atom, and a sulfur atom. Of these, the heteroatom is preferably an oxygen atom. When the ring structure represented by X is a cyclic hydrocarbon, examples of the cyclic hydrocarbon may include cycloalkane, cycloalkene, and aromatic hydrocarbon. In this case, examples of the ring structure represented by X may include cyclobutane, cyclopentane, cyclohexane, cyclopentene, cyclohexene, a benzene ring, a naphthalene ring, and an anthracene ring.

The alkyl group represented by R¹ is preferably an alkyl group containing 1 to 5 carbon atoms, more preferably an alkyl group containing 1 to 3 carbon atoms, further preferably an alkyl group having 1 or 2 carbon atoms, and particularly preferably a methyl group. In particular, R¹ is preferably a hydrogen atom or a methyl group.

Examples of the substituent represented by R may include substituents such as aliphatic hydrocarbon groups (e.g., an alkyl group, an alkenyl group, an alkynyl group, and a cycloalkyl group), aromatic hydrocarbon groups (e.g., a phenyl group, a naphthyl group, an anthryl group, a phenanthryl group, and a biphenyl group), a heterocyclic group, an acyl group, an alkoxy group, an amino group, a hydroxy group, a mercapto group, and a halogen atoms (e.g., a fluorine atom, a chlorine atom, a bromine atom, and an iodine atom). Otherwise, two Rs may together form an oxo group. The substituent may be substituted with another substituent, and examples of such other substituent may include the same as those described above. The substituent represented by R may be, for example, a hydrocarbon group, a halogen atom, or a monovalent group formed by combining a hydrocarbon group with one or more -O- or -C(=O)-. A monosaccharide structure may be linked to the substituent represented by R, and when the mediator contains two or more of the above-described ring structures in one molecule thereof, the above ring structure may be linked to the above substituent represented by R. When n is 2 or greater, the substituents represented by R may be linked to each other to form a ring.

Among others, the mediator is preferably a compound having the following structures:

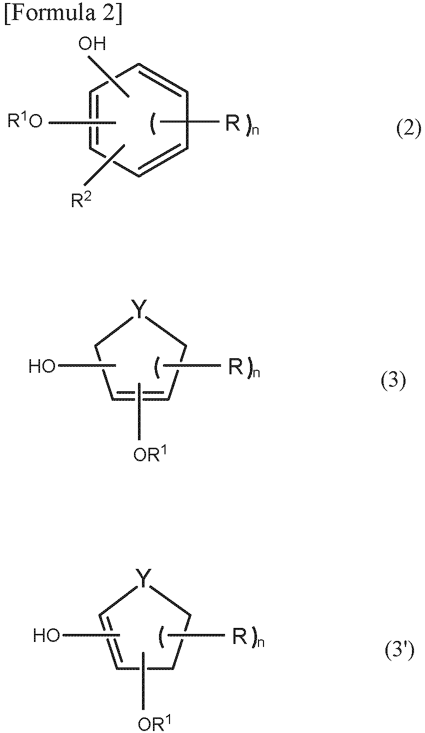

In the formula (2), R represents a substituent, n represents an integer from 0 to 3, R¹ represents a hydrogen atom or an alkyl group, and R² represents a hydrogen atom or a hydroxyl group. In addition, in the formulae (3) and (3'), R represents a substituent, n represents an integer from 0 to 2, R¹ represents a hydrogen atom or an alkyl group, and Y represents an oxygen atom, a nitrogen atom, or a sulfur atom. In the formulae (3) and (3'), Y is particularly preferably an oxygen atom, and the ring structure possessed by the mediator is particularly preferably a benzene ring or a dihydrofuran ring. Among others, when the ring structure possessed by the mediator is a dihydrofuran ring, it is preferable that the mediator has the structure represented by formula (3). In addition, when the ring structure possessed by the mediator is a dihydrofuran ring, it is preferable that the mediator has a hydroxyl group or an alkoxy group at positions 3 and 4 thereof.

In particular, the mediator is preferably a compound having the following structures:

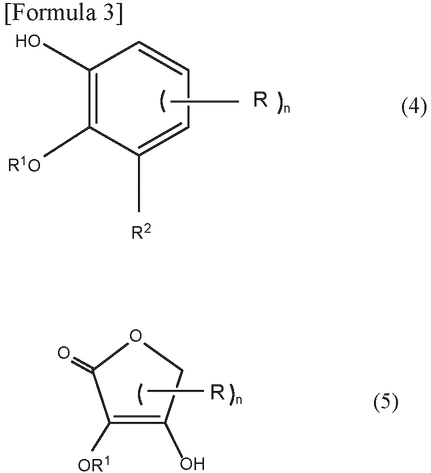

In the formula (4), R represents a substituent, n represents an integer from 0 to 3, R¹ represents a hydrogen atom or an alkyl group, and R² represents a hydrogen atom or a hydroxyl group. In the formula (4), n is more preferably an integer from 0 to 2, and particularly preferably an integer of 0 or 1. In addition, in the formula (5), R represents a substituent, n represents an integer of 0 or 1, and R¹ represents a hydrogen atom or an alkyl group. In the formula (5), n is particularly preferably 1.

Specific examples and preferred ranges of R¹ and R in the formulae (2) to (5) are the same as those of R¹ and R in the formula (1).

Specific examples of the mediator may include, for example, DL-catechin, tannic acid, chlorogenic acid, caffeic acid, curcumin, hesperidin, L-DOPA, pyrogallol, vanillin, eugenol, gallic acid monohydrate, ferulic acid, isoeugenol, α-glucosylrutin, rutin, lecithin, sugar beet pectin, resveratrol, anthocyanin, anthocyanidin, procyanidin, phloridzin, quercetin, vanillic acid, and apple polyphenol. Among them, the mediator is preferably at least one type selected from the group consisting of DL-catechin, tannic acid, chlorogenic acid, caffeic acid, curcumin, hesperidin, L-DOPA, pyrogallol, vanillin, eugenol, gallic acid monohydrate, ferulic acid, isoeugenol, α-glucosylrutin and rutin.

Among others, it is preferable that the mediator is a polyphenol. In the present description, polyphenols are compounds having a structure in which multiple hydroxyl groups bind to a benzene ring or a naphthalene ring. Examples of such polyphenols may include DL-catechin, tannic acid, chlorogenic acid, caffeic acid, L-DOPA, pyrogallol, gallic acid monohydrate, α-glucosylrutin, rutin, procyanidin, quercetin glycoside, verbascoside, isoverbascoside, diosmetin, diglucuronide, apigenin 7-diglucuronide, and luteolin 7-diglucuronide. Among these polyphenols, it is particularly preferable that the mediator is at least one type selected from the group consisting of α-glucosylrutin, chlorogenic acid, caffeic acid, tannic acid and rutin. By using the above-described compound as a mediator, the amount of phenylacetaldehyde produced can be increased more effectively.

Moreover, the mediator may also be a compound contained in a plant extract, and the mediator may be a compound contained, for example, in pectin, a green coffee bean extract, a tea extract, an apple extract, a lemon verbena extract, etc. Among them, the mediator is preferably a compound contained in at least one type selected from the group consisting of an apple extract, pectin, a green coffee bean extract, and a tea extract. For example, pectin contains ferulic acid; a green coffee bean extract and a tea extract contain DL-catechin, chlorogenic acid, and caffeic acid; an apple extract contains apple polyphenols such as procyanidins (a structure in which 2 to 15 catechins are bonded), catechins, chlorogenic acid, phlorizin, and quercetin glycoside; and a lemon verbena extract contains polyphenols such as verbascoside, isoverbascoside, diosmetin, diglucuronide, apigenin 7-diglucuronide, and luteolin 7-diglucuronide. These components and extracts may contain analogues of the above-mentioned compounds. Examples of the analogues may include compounds, which have the basic skeleton of the above-mentioned compounds, but in which at least one hydrogen atom in the side chain, etc. is substituted with a substituent. For example, examples of the analogue of chlorogenic acid may include 4,5-dicaffeoylquinic acid, 4-caffeoylquinic acid, 4-feruloylquinic acid, 3,5-dicaffeoylquinic acid, 3-caffeoylquinic acid, 3-feruloylquinic acid, 3,4-dicaffeoylquinic acid, 5-caffeoylquinic acid, and 5-feruloylquinic acid. Examples of the analogue of catechin may include epicatechin, epigallocatechin, and epigallocatechin gallate. Examples of the analogue of procyanidins may include procyanidin B1, procyanidin B2, and procyanidin A2. Also, pectin and the above-mentioned extracts may contain a plurality of the above-mentioned compounds or analogues.

A commercially available product can be used as an extract described above. For example, Sunfood 100 manufactured by Mitsubishi Chemical Corporation, the tea extract (food material) manufactured by SANCT Corporation, the apple polyphenol powder manufactured by FRONTIER FOODS CO., LTD., etc. can be used.

When a mediator is allowed to act on phenylalanine or an analogue thereof, the amount of the mediator to be acted on is not particularly limited, and the concentration of the mediator in the reaction solution is preferably 0.01 mM or more, more preferably 0.05 mM or more, further preferably 0.1 mM or more, and particularly preferably 0.5 mM or more. On the other hand, the concentration of the mediator in the reaction solution is preferably 500 mM or less, more preferably 300 mM or less, and further preferably 100 mM or less. Moreover, the concentration of the mediator in the reaction solution may be 50 mM or less, 25 mM or less, 10 mM or less, or 5 mM or less. Besides, the amount of the mediator to be added is preferably adjusted, as appropriate, depending on the type of the mediator.

The concentration of the mediator in the reaction solution is preferably 0.001% by mass or more, more preferably 0.005% by mass or more, further preferably 0.01% by mass or more, and particularly preferably 0.05% by mass or more. On the other hand, the concentration of the mediator in the reaction solution is preferably 50% by mass or less, more preferably 30% by mass or less, and further preferably 10% by mass or less. Moreover, the concentration of the mediator in the reaction solution may be 5% by mass or less, 2.5% by mass or less, 1% by mass or less, or 0.5% by mass or less. Besides, the amount of the mediator to be added is preferably adjusted, as appropriate, depending on the type of the mediator.

### < Multicopper oxidase >

The method for producing phenylacetaldehyde of the present invention preferably includes a step of allowing a mediator to act on phenylalanine or an analogue thereof in the presence of multicopper oxidase. Multicopper oxidase is an enzyme that contains 2 to 8 atoms of copper in the molecule thereof, which is necessary for enzyme activity, and is an enzyme that subjects oxygen to electron reduction so as to generate water molecules. Examples of such multicopper oxidase may include laccase, bilirubin oxidase, ascorbic acid oxidase, ceruloplasmin, Fet3p, CueO, CotA, stellacyanin, tyrosinase, catechol oxidase, and nitrite reductase.

In the step of producing phenylacetaldehyde of the present invention, it is considered that multicopper oxidase acts on a mediator containing a hydroxyl group to oxidize the hydroxyl group possessed by the ring structure of the mediator, and that the oxidized compound acts on phenylalanine or an analogue thereof to cause Strecker degradation, resulting in generation of phenylacetaldehyde.

Among others, it is preferable that the multicopper oxidase is at least one type selected from phenol oxidase and bilirubin oxidase. It is more preferable that the multicopper oxidase is phenol oxidase, and the phenol oxidase is preferably polyphenol oxidase, and among these, the phenol oxidase is particularly preferably laccase.

Laccase is an enzyme (EC 1.10.3.2) having phenol oxidase activity, and can be used in any given application that utilizes the oxidation reaction of a substrate and/or a variety of accompanying chemical reactions caused by radical species of reaction intermediates generated by the aforementioned oxidation reaction. Examples of such oxidation reaction or accompanying chemical reactions may include: the oxidation of phenolic compounds such as o-, p-diphenol, urushiol, and laccol; the oxidation of aromatic amines such as p-phenylenediamine; the decomposition of lignin; and the crosslinking of proteins having easily oxidized functional groups such as tyrosine side chains (phenolic hydroxyl groups), cysteine side chains (sulfhydryl groups), lysine side chains (ε-amino groups), and histidine side chains (imidazole groups). The chemical modification of a substrate by the oxidation reaction and accompanying chemical reactions of laccase in this manner can also be called "oxidative modification."

Laccase is present in plants, fungi, bacteria, and animals. In particular, the laccase used in the present invention is preferably derived from fungi or bacteria. Specific examples may include laccases derived from Aspergillus sp., Neurospora sp., Podospora sp., Botrytis sp., Collybia sp., Fomes sp., Lentinus sp., Pleurotus sp., Pycnoporus sp., Pyricularia sp., Trametes sp., Rhizoctonia sp., Rigidoporus sp., Coprinus sp., Psatyrella sp., Myceliophtera sp., Schtalidium sp., Polyporus sp., Phlebia sp., Coriolus sp., and Bacillus sp. Among other, laccase derived from Trametes sp. is preferably used. By using the laccase derived from Trametes sp., phenylacetaldehyde can be efficiently produced.

Laccase can be prepared from the culture medium of the aforementioned microorganisms serving as origins of laccase. A specific preparation method may be a method of recovering laccase from the culture medium or cell mass of the above-described microorganism. For example, when laccase-secreting microorganisms are used, after the cell mass has been recovered in advance from the culture medium by filtration, centrifugation, etc., as necessary, the enzyme is then separated and/or purified. On the other hand, when non-laccase-secreting microorganisms are used, after the cell mass has been recovered in advance from the culture medium, as necessary, the cell mass is disintegrated by a pressure treatment, an ultrasonic treatment, etc., so that the enzyme is exposed, and the enzyme is then separated and/or purified. The method of separating and/or purifying the enzyme is not particularly limited, and a known protein separation and/or purification method can be adopted. Examples of the protein separation and/or purification method may include a centrifugation method, a UF concentration method, a salting-out method, and various chromatographic methods using ion exchange resins, etc. The separated and/or purified enzyme can be pulverized by drying methods such as freeze-drying and vacuum drying, or can also be pulverized by using appropriate excipients and/or drying aids in the drying methods. In addition, the separated and/or purified enzymes can also be liquefied by adding appropriate additives and sterilizing by filtration.

As laccase, a commercially available product can also be used. An example of a preferred commercially available product may be Trametes sp.-derived laccase (LC), which is manufactured by Amano Enzyme, Inc.

Bilirubin oxidase is an enzyme (EC1.3.3.5) having bilirubin oxidase activity. Bilirubin oxidase derived from, for example, microorganisms belonging to Myrothecium sp., Coprinus sp., Penicillium sp., or Bacillus sp. can be used. Examples of the Myrothecium sp. may include preserved strains such as *Myrothecium verrucaria* MT-1, FERM-BP 653 (see Agricultural and Biological Chemistry, Vol. 45, pp. 2383-2384 (1981)), *Myrothecium verrucaria* IFO 6113, *Myrothecium verrucaria* IFO 6133, *Myrothecium verrucaria* IFO 6351, *Myrothecium verrucaria* IFO 9056, *Myrothecium tinctum* IFO 9950, and *Myrothecium verrucaria* IFO 9531. Examples of the Coprinus sp. may include preserved strains such as *Coprinus cinereus* IFO 8371 and *Coprinus lagopides* IFO 30120. An example of the Penicillium sp. may be *Penicillium jensinerum* (see JP Patent Publication No. 63-309187 A (1988)). An example of the Bacillus sp. may be *Bacillus licheniformis* (see JP Patent Publication No. 61-209587 A (1986). These strains are subjected to liquid culture or solid culture by conventional methods, and the culture medium is then subjected to extraction, salting out, dialysis, ion exchange, gel filtration, etc. to obtain purified preparations of the enzyme bilirubin oxidase. On the other hand, the enzyme bilirubin oxidases derived from *Schizophyllum commune* (see JP Patent Publication No. 59-135886 A (1984)), derived from Asteraceae plants (see JP Patent Publication No. 62-285782 A (1987)), and derived from alfalfa (see JP Patent Publication No. 6-319536 A (1994)), as well as recombinants (see JP Patent Publication No. 5-199882 A (1993)), are also known, and these bilirubin oxidases can also be used.

As bilirubin oxidase, a commercially available product can also be used. As examples of preferred commercially available products, commercially available products manufactured by Amano Enzyme, Inc., Takara Bio, Inc., Asahi Kasei Corporation, Sigma-Aldrich Corporation, etc. may be used.

When multicopper oxidase is allowed to act on a mediator in the step of producing phenylacetaldehyde, the amount of multicopper oxidase to be acted on is not particularly limited, and it is preferable to add multicopper oxidase, so that the enzyme activity becomes, for example, 1 U/mL or more relative to 1 mM of the mediator. From the viewpoint of further increasing the amount of phenylacetaldehyde generated, the enzyme activity is more preferably 5 U/mL or more, even more preferably 10 U/mL or more, further preferably 12 U/mL or more, and particularly preferably 20 U/mL or more, relative to 1 mM of the mediator. Moreover, the enzyme activity may also be 40 U/mL or more, 60 U/mL or more, 80 U/mL or more, 100 U/mL, or 110 U/mL or more. The upper limit value of the enzyme activity is not particularly limited, and it is preferably 2000 U/mL or less, more preferably 1500 U/mL or less, further preferably 1200 U/mL or more, and particularly preferably 1000 U/mL or less, relative to 1 mM of the mediator. Furthermore, the upper limit value of the enzyme activity may be 800 U/mL or less, 600 U/mL or less, 400 U/mL or less, 300 U/mL or less, or 200 U/mL or less. When laccase is used as such multicopper oxidase, the additive amount of laccase is preferably within the above-described range. Besides, the additive amount is preferably adjusted, as appropriate, depending on the type of multicopper oxidase.

The amount of multicopper oxidase to be acted on a mediator in the step of producing phenylacetaldehyde is not particularly limited, and it is preferable to add multicopper oxidase, so that the enzyme activity becomes, for example, 1 U/mL or more per 10 mM of phenylalanine. From the viewpoint of further increasing the amount of phenylacetaldehyde generated, the enzyme activity is more preferably 5 U/mL or more, even more preferably 10 U/mL or more, further preferably 12 U/mL or more, and particularly preferably 20 U/mL or more, relative to 10 mM phenylalanine. Moreover, the enzyme activity may also be 40 U/mL or more, 60 U/mL or more, 80 U/mL or more, 100 U/mL, or 110 U/mL or more. The upper limit value of the enzyme activity is not particularly limited, and it is preferably 2000 U/mL or less, more preferably 1500 U/mL or less, further preferably 1200 U/mL or less, and particularly preferably 1000 U/mL or less, relative to 10 mM phenylalanine. Furthermore, the upper limit value of the enzyme activity may be 800 U/mL or less, 6000 U/mL or less, 400 U/mL or less, 300 U/mL or less, or 200 U/mL or less. When laccase is used as such multicopper oxidase, the additive amount of laccase is preferably within the above-described range. Besides, the additive amount of laccase is preferably adjusted, as appropriate, depending on the type of multicopper oxidase.

The enzyme activity of multicopper oxidase is measured by the following method. First, a 0.25 mol/L phenol test solution, a 0.009 mol/L 4-aminoantipyrine test solution, and a 1 mol/L acetic acid/sodium acetate buffer (pH 4.5) are mixed with one another at a ratio of 1 : 1 : 0.5 to prepare a substrate solution. This substrate solution (2.5 ml) is placed in a cuvette and is then preheated at 30°C, and thereafter, a 0.5 ml of enzyme solution is added and stirred, and is then incubated at 30°C. The absorbance at 505 nm is measured after 10 seconds and 40 seconds. When the absorbance increases by 0.1 per minute, the amount of enzyme contained in 1 mL of the reaction solution is defined as 1 unit (U).

### < Optional components >

In the step of producing phenylacetaldehyde, in addition to the above-mentioned components, optional components may be present, as appropriate. For example, a pH adjuster (hydrochloric acid, sodium hydroxide, citric acid, acetic acid, etc.) may be added as an optional component.

### < Production conditions >

The reaction time, the temperature, the pH of the reaction solution, and the like, which are applied when the mediator is allowed to act on phenylalanine or an analogue thereof, are not particularly limited. The reaction temperature is, for example, preferably 10°C or higher, more preferably 15°C or higher, further preferably 20°C or higher, and particularly preferably 25°C or higher. On the other hand, the reaction temperature is preferably 80°C or lower, more preferably 70°C or lower, and further preferably 60°C or lower. In addition, the reaction temperature may be 50°C or lower, or may be 40°C or lower. The pH of the reaction solution is, for example, preferably pH 4 or more, and more preferably pH 5 or more. On the other hand, the pH of the reaction solution is preferably pH 10 or less, more preferably pH 9 or less, and further preferably pH 8 or less. The reaction time is, for example, preferably 1 minute or more, more preferably 10 minutes or more, and further preferably 15 minutes or more. On the other hand, the reaction time is preferably 240 hours or less, more preferably 96 hours or less, further preferably 48 hours or less, and particularly preferably 24 hours or less. Moreover, the reaction time may be 12 hours or less, 6 hours or less, or 3 hours or less. Besides, even in a case where multicopper oxidase is present when the mediator is allowed to act on phenylalanine or an analogue thereof, the above-described reaction conditions are preferably adopted. By adopting the above-described reaction conditions, phenylacetaldehyde can be produced with good efficiency. Besides, the optimal reaction conditions may be determined through preliminary experiments, etc.

In the step of allowing the mediator to act on phenylalanine or an analogue thereof, a shaking treatment or a stirring treatment may be carried out during at least a portion of the above-described reaction time. In the shaking treatment or the stirring treatment, it is preferable to carry out shaking or stirring under conditions in which individual components are sufficiently mixed with one another.

When both the multicopper oxidase and the mediator are allowed to act on phenylalanine or an analogue thereof, it is preferable to add the multicopper oxidase and the mediator to a solution containing phenylalanine. At this time, the order of addition is not particularly limited. The multicopper oxidase may be added to a solution containing phenylalanine, and then the mediator may be added thereto, or the mediator may be added to a solution containing phenylalanine, and then the multicopper oxidase may be added thereto. Otherwise, the multicopper oxidase and the mediator may be simultaneously added to a solution containing phenylalanine.

As a solvent used when the multicopper oxidase and the mediator are allowed to act on phenylalanine or an analogue thereof, water, an organic solvent, or a mixed solvent of these is preferably used. Examples of the organic solvent may include: alcohols (aliphatic saturated alcohols, aliphatic unsaturated alcohols, alicyclic alcohols, aromatic alcohols, heterocyclic alcohols, etc., such as methyl alcohol, ethyl alcohol, propyl alcohol, isopropyl alcohol, butyl alcohol, isobutyl alcohol, amyl alcohol, hexyl alcohol, heptyl alcohol, octyl alcohol, capryl alcohol, allyl alcohol, cyclopentanol, and benzyl alcohol); ethers (methyl ether, ethyl ether, propyl ether, methyl ethyl ether, methyl propyl ether, methyl butyl ether, vinyl ether, etc.); esters (esters of carboxylic acids such as formic acid, acetic acid, propionic acid, butyric acid, valeric acid and phthalic acid, with alcohols such as methyl, ethyl, propyl, isopropyl, butyl, isobutyl, n-amyl and isoamyl); and ketones (aliphatic saturated ketones, aliphatic unsaturated ketones, alicyclic ketones, aromatic ketones, etc., such as acetone, ethyl methyl ketone, methyl propyl ketone, methyl vinyl ketone, cyclobutanone, and acetophenone). However, from the viewpoint of reducing burden on the environment, the reaction solvent is preferably an aqueous solvent.

After the step of allowing multicopper oxidase and a mediator to act on phenylalanine or an analogue thereof, the method for producing phenylacetaldehyde may include a step of inactivating the multicopper oxidase. **In** the step of inactivating the multicopper oxidase, for example, the reaction solution containing the multicopper oxidase can be heated to 80°C or higher, or the enzyme solution can be isolated by centrifugation or the like.

The amount of phenylacetaldehyde generated can be analyzed, for example, using GC-MS. **In** addition, the degree of phenylacetaldehyde generation can also be evaluated by sensory evaluation of the presence or absence of a floral scent.

### (Phenylacetaldehyde)

The present invention may relate to phenylacetaldehyde produced by the aforementioned production method. Since the phenylacetaldehyde obtained by the aforementioned production method is safe, it may be added to pharmaceutical products or food products and beverages. That is to say, the present invention may relate to phenylacetaldehyde used in pharmaceutical products or phenylacetaldehyde used in food products and beverages, or may also relate to a pharmaceutical product, or a food product and a beverage, containing the phenylacetaldehyde obtained by the aforementioned production method.

The phenylacetaldehyde produced by the aforementioned production method may be isolated, for example, by dissolving it in an organic solvent, etc., which dissolves phenylacetaldehyde, separating the organic solvent layer, and then distilling away the solvent.

### (Phenylacetaldehyde-generating enzyme agent/enzyme agent for improving flavor of food products and beverages)

The present invention may relate to a phenylacetaldehyde-generating enzyme agent, containing a mediator and multicopper oxidase. **In** addition, the present invention may also relate to an enzyme agent for improving the flavor of food products and beverages, containing a mediator and multicopper oxidase. Herein, the mediator is a compound containing a ring structure having at least one hydroxyl group. By adding the phenylacetaldehyde obtained by the aforementioned production method to food products and beverages or to materials for food products and beverages, off-flavors are masked, a rich taste or a scent is added to the food products and beverages, so that the flavor can be improved.

The phenylacetaldehyde-generating enzyme agent and the enzyme agent for improving the flavor of food products and beverages may be in any form, such as powders, a solid, a gel, or a liquid. Phenylacetaldehyde can be generated by adding the phenylacetaldehyde-generating enzyme agent or the enzyme agent for improving the flavor of food products and beverages to a solution containing phenylalanine. Alternatively, the phenylacetaldehyde-generating enzyme agent or the enzyme agent for improving the flavor of food products and beverages may also be added to food products and beverages containing phenylalanine or to materials for food products and beverages, and in such a case, phenylacetaldehyde can be generated from the phenylalanine contained in the food products and beverages or the materials for food products and beverages. It is to be noted that if food products and beverages or materials for food products and beverages originally contain the above-described mediator, it may not be necessary to add the mediator in some cases.

### (Method for improving flavor of food products and beverages)

The present invention may relate to a method for improving the flavor of food products and beverages, including a step of allowing a mediator and multicopper oxidase to act on phenylalanine or an analogue thereof. Herein, the mediator is a compound containing a ring structure having at least one hydroxyl group. When food products and beverages or materials for food products and beverages materials contain phenylalanine, the method for improving the flavor of food products and beverages may be a method for improving the flavor of food products and beverages, including a step of allowing a mediator and multicopper oxidase to act on the food products and beverages or the food products and beverages materials, or may also be a method for improving the flavor of food products and beverages, including a step of further adding phenylalanine to the food products and beverages or the food products and beverages materials and then allowing a mediator and multicopper oxidase to act on the food products and beverages. By allowing such a mediator and multicopper oxidase to act on phenylalanine or an analogue thereof, phenylacetaldehyde is generated, and the generated phenylacetaldehyde masks off-flavors or adds a rich taste or a scent to food products and beverages, so that the flavor of the food products and beverage can be improved.

Examples of the food products and beverages may include: beverages such as dairy products, coffee drinks, black tea drinks, green tea drinks, beer, plant-based drinks (soy milk, almond milk, oat milk, rice milk, coconut milk, pea milk, and potato milk), wine and cocoa; and plant-based meat substitutes. Moreover, the materials for food products and beverages are the raw materials (materials) used to produce food products and beverages. Examples of the materials for food products and beverages may include: cereal crops, such as barley, rice, wheat, rye, oats, buckwheat, sorghum, barnyard millet, millet, teff, quinoa, and corn; pulses, such as soybeans, lentils, broad beans, peas, chickpeas, mung beans, lupin beans, and kidney beans; nuts, such as hemp (industrial hemp), canary seeds, linseeds, almonds, cashew nuts, hazelnuts, pecan nuts, macadamia nuts, pistachios, walnuts, Brazil nuts, peanuts, coconuts, pili nuts, chestnuts, sesame seeds, and pine nuts; and tubers, such as potatoes, sweet potatoes, and taro.

### Examples

Hereinafter, the characteristics of the present invention will be more specifically described in the following examples and comparative examples. The materials, amounts used, proportions, treatment contents, treatment procedures, etc. shown in the following examples may be changed, as appropriate, without deviating from the gist of the present invention. Therefore, the scope of the present invention should not be interpreted limitedly by the specific examples shown below.

### (Method of measuring enzyme activity)

The reagents described in the present measurement method were prepared based on the polyphenol oxidase activity measurement method in the 9th edition of the Japan's Specifications and Standards for Food Additives (JSFA). Specifically, a 0.25 mol/L phenol test solution, a 0.009 mol/L 4-aminoantipyrine test solution, and a 1 mol/L acetic acid/sodium acetate buffer (pH 4.5) were mixed with one another at a ratio of 1 : 1 : 0.5 to prepare a substrate solution. This substrate solution (2.5 ml) was placed in a cuvette and was then preheated at 30°C, and thereafter, a 0.5 ml of enzyme solution was added and stirred, and was then incubated at 30°C. The absorbance at 505 nm was measured after 10 seconds and 40 seconds. When the absorbance increased by 0.1 per minute under these conditions, the amount of enzyme contained in 1 mL of the reaction solution was defined as 1 unit (U).

### (Materials used)

L-phenylalanine (Phe) (FUJIFILM Wako Pure Chemical Corporation) was used as a substrate (oxidation substrate). In addition, as enzymes, the laccase (LC) derived from Trametes sp. (Amano Enzyme, Inc.) and the bilirubin oxidase (BO) derived from Myrothecium sp. (Amano Enzyme, Inc.) were used. Besides, the compounds shown in the table below were used as mediators (reducing substrates).

**[Table 1]**

| Substance name | Manufacturer |
|---|---|
| DL-catechin | Tokyo Chemical Industry Co., Ltd. |
| Tannic acid | FUJIFILM Wako Pure Chemical Corporation |
| Rutin | FUJIFILM Wako Pure Chemical Corporation |
| Chlorogenic acid | Tokyo Chemical Industry Co., Ltd. |
| Caffeic acid | Tokyo Chemical Industry Co., Ltd. |
| Curcumin | FUJIFILM Wako Pure Chemical Corporation |
| Hesperidin | FUJIFILM Wako Pure Chemical Corporation |
| L-DOPA | Sigma-Aldrich |
| Pyrogallol | FUJIFILM Wako Pure Chemical Corporation |
| GENU pectin type BETA BI-J (pectin)* | Sansho Co., Ltd. |
| Vanillin | FUJIFILM Wako Pure Chemical Corporation |
| Eugenol | Tokyo Chemical Industry Co., Ltd. |
| Gallic Acid Monohydrate | FUJIFILM Wako Pure Chemical Corporation |
| Ferulic Acid | Sigma-Aldrich |
| Isoeugenol | FUJIFILM Wako Pure Chemical Corporation |
| Green Coffee Bean Extract P* | Oryza Oil & Fat Chemical Co., Ltd. |
| Sunfood 100 (tea extract)* | Mitsubishi Chemical Corporation |
| Butylated hydroxyanisole | Sigma-Aldrich |
| α-Glucosylrutin | FUJIFILM Wako Pure Chemical Corporation |
| L-Ascorbic acid | FUJIFILM Wako Pure Chemical Corporation |
| Sodium erythorbate monohydrate | FUJIFILM Wako Pure Chemical Corporation |
| Apple polyphenol powder * | FRONTIER FOODS CO., LTD. |

The mediator substances with the symbol * were each added to a final concentration of 0.1% by mass. The other mediators were each added to a final concentration of 1 mM.

### (Test Example 1)

To a 15-ml centrifuge tube, 1 ml of 250 mM Tris-HCl (pH 7.0) buffer, 2.5 ml of 20 mM L-phenylalanine, 0.5 ml of 10 mM or 1 mass% each type of mediator solution, and 1 ml of 0.5 mass% laccase solution were added, and thereafter, the reaction was carried out by shaking at 30°C for 2 hours. After completion of the reaction, the enzyme was inactivated by a boiling treatment for 10 minutes. It is to be noted that the mediator was dissolved in water or ethanol and was then added to a final concentration of 1 mM or 0.1 mass%.

**[Table 2]**

| Reaction composition | Additive amount (ml) | Final concentration |
|---|---|---|
| 250 mM Tris-HCl Buffer (pH 7.0) | 1 | 50 mM |
| 10 mM or 1 mass% mediator | 0.5 | 1 mM or 0.1 mass% |
| 20 mM L-phenylalanine | 2.5 | 10 mM |
| 0.5 mass% laccase solution | 1 | 0.1 mass% (120 U/mL) |

After 0.8 g of NaCl had been dissolved in 4 ml of the reaction solution, solid-phase extraction was carried out using SPME Arrow, and generation of phenylacetaldehyde (PAA) was then analyzed by GC-MS.

The analysis conditions for PAA were as follows, and the concentration of PAA was calculated using a calibration curve.

### SPME Arrow (solid-phase extraction)

Fiber used: DVB/PDMS/CAR, 1 cm
Extraction conditions: 50°C, 30 minutes of incubation → 50°C, 30 minutes of adsorption → 240°C, 1 minute of desorption
Bake conditions: 250°C, 30 minutes

### GC-MS QP-2020NX

Column: InertCap pure-WAX (30 m, 0.25 mm, 0.25 µm)
GC conditions: Temperature increase: 60°C, 2 min, 10°C/min to 170°C, 20°C/min to 240°C, 240°C 10 min injection port: 250°C
MS conditions: Measurement mode: SIM, Monitor ion m/z 65, 91, 120

The sensory evaluation shown in Table 3 was carried out by confirming the presence or absence of the floral scent characteristic of phenylacetaldehyde after completion of the reaction, and the odor was evaluated based on the following indicators.
o: Has a floral odor
Δ: Has a faint floral odor
×: Does not have a floral odor

**[Table 3]**

| | | Mediator | Structural formula | PAA (ppm) | Sensory evaluation |
|---|---|---|---|---|---|
| Comp. Ex. | 1 | Ethanol | CH₃CH₂OH | 0.0 | × |
| Comp. Ex. | 2 | Water | H₂O | 0.0 | × |
| Ex. | 1 | α-Glucosylrutin | 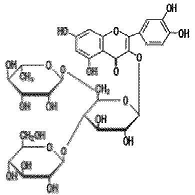 | 21.1 | o |
| Ex. | 2 | Chlorogenic Acid | 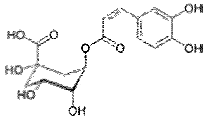 | 10.7 | o |
| Ex. | 3 | Caffeic Acid | 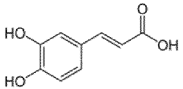 | 6.5 | o |
| Ex. | 4 | Green coffee bean extract | containing chlorogenic acid, ferulic acid, caffeic acid, dicaffeoylquinic acid, etc. | 17.7 | o |
| Ex. | 5 | Tannic acid | 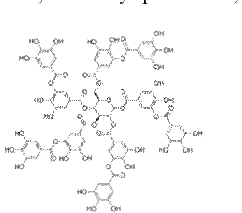 | 7.0 | o |
| Ex. | 6 | Rutin | 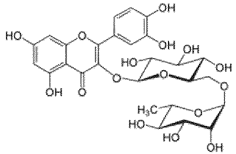 | 5.7 | o |
| Ex. | 7 | DL-catechin | 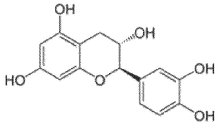 | 1.6 | o |
| | | | e.g.: (+)-catechin | | |
| Ex. | 8 | Sunfood 100 (tea extract) | containing catechin, etc. | 5.6 | o |
| Ex. | 9 | Gallic acid monohydrate | 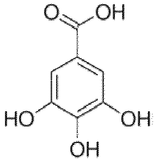 | 3.7 | o |
| Ex. | 10 | Pyrogallol | 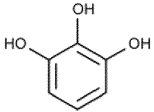 | 2.6 | o |
| Ex. | 11 | L-dopa | 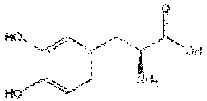 | 1.3 | o |
| Ex. | 12 | Curcumin | 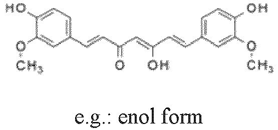 | 0.3 | o |
| Ex. | 13 | Ferulic acid | 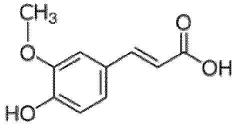 | 0.3 | Δ |
| Ex. | 14 | Pectin | containing ferulic acid, etc. | 0.1 | Δ |
| Ex. | 15 | Vanillin | 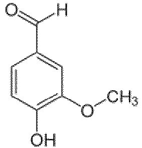 | 0.3 | Δ |
| Ex. | 16 | Isoeugenol | 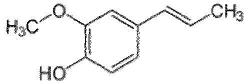 | 0.3 | Δ |
| Ex. | 17 | Eugenol | 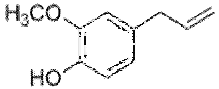 | 0.2 | Δ |
| Ex. | 18 | Hesperidin | 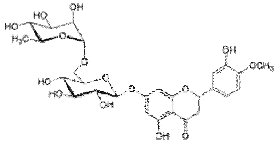 | 0.2 | Δ |
| Ex. | 19 | Butylated hydroxyanisole | 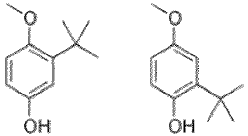 | 0.2 | Δ |

**[Table 4]**

| | | Mediator | Structural formula | PAA (ppm) |
|---|---|---|---|---|
| Comp. Ex. | 1 | Ethanol | CH₃CH₂OH | 0.0 |
| Comp. Ex. | 2 | Water | H₂O | 0.0 |
| Ex. | 20 | L-Ascorbic acid | 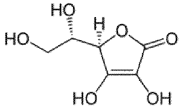 | 0.3 |
| Ex. | 21 | Sodium erythorbate monohydrate | 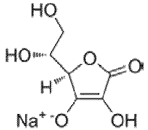 | 0.1 |

From the above-described results, it was found that compounds containing a ring structure having at least one hydroxyl group tend to generate PAA. It was found that, in particular, compounds containing a catechol structure tend to generate PAA.

### (Test Example 2)

A test was conducted using Myrothecium sp.-derived bilirubin oxidase (BO) (Amano Enzyme, Inc.) as an enzyme. The reaction composition was as shown in Table 5 below. Specifically, 1 ml of 250 mM Tris-HCl (pH 7.0) buffer, 2.5 ml of 20 mM L-phenylalanine, 0.5 ml of 10 mM DL-catechin, and 1 ml of 0.5 mass% enzyme (Trametes sp.-derived laccase (LC) or Myrothecium sp.-derived bilirubin oxidase(BO)) were added to a 15 ml centrifuge tube, and thereafter, the reaction was carried out by shaking at 30°C for 2 hours. After completion of the reaction, an equal amount (5 ml) of 0.8M formic acid solution was added to the reaction solution so as to terminate the reaction. Thereafter, 0.8 g of NaCl was dissolved in 4 ml of the reaction solution, solid-phase extraction was then carried out using SPME Arrow, and generation of phenylacetaldehyde (PAA) was then analyzed by GC-MS. Moreover, a sensory evaluation was carried out in terms of the presence or absence of the floral scent characteristic of phenylacetaldehyde by the same method as that in Test Example 1.

**[Table 5]**

| Reaction composition | Additive amount (ml) | Final concentration |
|---|---|---|
| 250 mM Tris-HCl Buffer (pH 7.0) | 1 | 50 mM |
| 10 mM DL-Catechin in ethanol | 0.5 | 1 mM |
| 20 mM L-Phenylalanine | 2.5 | 10 mM |
| 0.5 mass% Enzyme (LC or BO) | 1 | 0.1 mass% (120 U/mL) |

**[Table 6]**

| Results | | | | | |
|---|---|---|---|---|---|
| | | Enzyme | Mediator | PAA (ppm) | Sensory evaluation |
| Comp. Ex. | 11 | Non | Ethanol | 0.0 | × |
| Ex. | 31 | LC | DL-Catechin | 8.1 | o |
| Comp. Ex. | 12 | LC | Ethanol | 0.0 | × |
| Ex. | 32 | BO | DL-Catechin | 7.7 | o |
| Comp. Ex. | 13 | BO | Ethanol | 0.0 | × |

From the above-described results, it was confirmed that even when bilirubin oxidase derived from Myrothecium sp. was used as an enzyme, almost the same amount of PAA was generated as when laccase was used.

### (Test Example 3)

The influence of the pH of the reaction solution was confirmed. The reaction composition was as shown in Table 7 below. Specifically, 2 ml of 250 mM Tris-HCl (pH 7.0) buffer, or phosphate buffer (pH 6.0) or citrate buffer (pH 5.0), 5 ml of 20 mM L-phenylalanine, 1 ml of 10 mM tannic acid solution, and 2 ml of 0.5 mass% laccase solution were added to a 15 ml centrifuge tube, and thereafter, the reaction was carried out by shaking at 30°C or 50°C for 0.5 to 4 hours. Thereafter, the enzyme was inactivated by a boiling treatment for 10 minutes.

After 0.8 g of NaCl had been dissolved in 4 ml of the reaction solution, solid-phase extraction was carried out using SPME Arrow, and generation of phenylacetaldehyde (PAA) was then analyzed by GC-MS.

**[Table 7]**

| Reaction composition | Additive amount (ml) | Final concentration |
|---|---|---|
| 250 mM Tris-HCl Buffer (pH 7.0) | 2 | 50 mM |
| 250 mM Phosphate Buffer (pH 6.0) | | |
| 250 mM Citrate Buffer (pH 5.0) | | |
| 10 mM Tannic acid | 1 | 1 mM |
| 20 mM L-Phenylalanine | 5 | 10 mM |
| 0.5 mass% Laccase solution | 2 | 0.1 mass% (120 U/mL) |

**[Table 8]**

| Results | | | | |
|---|---|---|---|---|
| Example | Reaction time (h) | Reaction temperature | pH | PAA (ppm) |
| 41 | 0.5 | 30°C | 7.0 | 1.5 |
| 42 | 1 | | | 3.1 |
| 43 | 2 | | | 4.2 |
| 44 | 4 | | | 5.5 |
| 45 | 0.5 | 50°C | 7.0 | 4.1 |
| 46 | 1 | | | 6.1 |
| 47 | 2 | | | 9.1 |
| 48 | 4 | | | 10.9 |
| 49 | 2 | 30°C | 5.0 | 6.1 |
| 50 | | | 6.0 | 5.8 |
| 51 | | | 7.0 | 4.9 |
| 52 | 2 | 50°C | 5.0 | 15.7 |
| 53 | | | 6.0 | 21.4 |
| 54 | | | 7.0 | 8.8 |

Regarding the reaction time, for 0.5 hours, PAA was produced at a level that was detectable in the sensory evaluation. Regarding the reaction temperature, generation of PAA was observed even at 30°C, but the amount of PAA generated was larger at 50°C. Regarding the pH, generation of PAA was observed under all pH conditions, but the preferred condition was pH 6 or less.

### (Test Example 4)

The effects of a case of not using enzymes was confirmed using a green coffee bean extract, α-glucosylrutin and tannic acid. The reaction composition was as shown in Table 9 below. Specifically, 1 ml of 250 mM Tris-HCl (pH 7.0) buffer or phosphate buffer (pH 6.0), 2.5 ml of 20 mM L-phenylalanine, 0.5 ml of 10 mM or 1 mass% mediator, and 1 ml of 0.5 mass% laccase solution were added to a 15 ml centrifuge tube, and thereafter, the reaction was carried out by shaking at 30°C or 50°C for 2 hours. After that, the enzyme was inactivated by a boiling treatment for 10 minutes. As mediators used herein, the green coffee bean extract was added to a final concentration of 0.1% by mass, and the tannic acid and the α-glucosylrutin were each added to a final concentration of 1 mM.

After 0.8 g of NaCl had been dissolved in 4 ml of the reaction solution, solid-phase extraction was carried out using SPME Arrow, and generation of phenylacetaldehyde (PAA) was then analyzed by GC-MS.

**[Table 9]**

| Reaction composition | Additive amount (ml) | Final concentration |
|---|---|---|
| 250 mM Tris-HCl Buffer (pH7.0) or 250 mM Phosphate Buffer(pH6.0) | 1 | 50 mM |
| 10mM or 1 mass% Mediator | 0.5 | 1 mM or 0.1 mass% |
| 20mM L-Phenylalanine | 2.5 | 10 mM |
| 0.5 mass% Laccase solution | 1 | 0.1 mass% (120 U/mL) |

**[Table 10]**

| Results | | | | | | |
|---|---|---|---|---|---|---|
| | | Mediator | Enzyme | Reaction temperature | pH | PPA (ppm) |
| Comp. Ex. | 21 | Water | No | 30 | 7 | 0.1 |
| Comp. Ex. | 22 | | Yes | | | 0 |
| Ex. | 61 | Tannic acid | No | | | 0.2 |
| Ex. | 62 | | Yes | | | 5.2 |
| Ex. | 63 | Green coffee bean extract | No | | | 0.5 |
| Ex. | 64 | | Yes | | | 13.5 |
| Ex. | 65 | α-Glucosylrutin | No | | | 0.4 |
| Ex. | 66 | | Yes | | | 13.8 |
| Comp. Ex. | 23 | Water | No | 50 | 6 | 0.1 |
| Comp. Ex. | 24 | | Yes | | | 0.1 |
| Ex. | 67 | Tannic acid | No | | | 1.7 |
| Ex. | 68 | | Yes | | | 25.4 |
| Ex. | 69 | Green coffee bean extract | No | | | 2.1 |
| Ex. | 70 | | Yes | | | 6.6 |
| Ex. | 71 | α-Glucosylrutin | No | | | 0.8 |
| Ex. | 72 | | Yes | | | 9.1 |

Even in the case of not using enzymes, generation of PAA was observed, but it was confirmed that, in the presence of enzymes, generation of PAA tended to further increase.

### (Test Example 5)

The same test as above was carried out with the exception that soy milk was used instead of the Tris-HCl buffer in the aforementioned test examples. Generation of PAA was observed even when the soy milk was used.

### (Test Example 6)

1 ml of 250 mM phosphate buffer (pH 6.0), 2.5 ml of 20 mM L-phenylalanine, 0.5 ml of 1 mass% apple polyphenol solution, and 1 ml of 0.5 mass% laccase solution were added to a 15 ml centrifuge tube, and thereafter, the reaction was carried out by shaking at 50°C for 1 hour. After completion of the reaction, the enzyme was inactivated by a boiling treatment for 10 minutes.

**[Table 11]**

| Reaction composition | Additive amount (ml) | Final concentration |
|---|---|---|
| 250 mM Phosphate buffer (pH 6.0) | 1 | 50 mM |
| 1 mass% Apple polyphenol solution | 0.5 | 0.1 mass% |
| 20 mM L-Phenylalanine | 2.5 | 10 mM |
| 0.5 mass% Laccase solution | 1 | 0.1 mass% (120 U/mL) |

After 0.8 g of NaCl had been dissolved in 4 ml of the reaction solution, solid-phase extraction was carried out using SPME Arrow, and the generated products were then analyzed by GC-MS. The analysis conditions are as follows.

### SPME Arrow (solid-phase extraction) common

Fiber used: DVB/PDMS/CAR, 1cm
Extraction conditions: 50°C, 30 minutes incubation → 50°C, 30 minutes adsorption → 240°C, 1 minute desorption
Bake conditions: 250°C, 30 minutes

### GC-MS QP-2020NX

Column: InertCap pure-WAX (30 m, 0.25 mm, 0.25 µm)
GC conditions: Temperature increase: 40°C 4 min, 10°C/min to 170°C, 20°C/min to 240°C, 240°C 10 min injection port: 250°C
MS conditions: Measurement mode SCAN

### < Sensory evaluation >

The products contained in the reaction solution were evaluated in terms of odor. As a result, a strong floral scent was detected.

### < GC-MS analysis >

The products were identified using the standards shown in Table 12 below.

**[Table 12]**

| Type | Substance name | Manufacturer |
|---|---|---|
| Product | Phenylacetaldehyde (PAA) | Combi-Blocks |
| Product | Benzaldehyde (BA) | Sigma-Aldrich |

**[Table 13]**

| Amino acid | Main products appeared |
|---|---|
| L-Phenylalanine | Benzaldehyde, phenylacetaldehyde |

As a result of the GC-MS analysis, as shown in Table 13, it was confirmed that in addition to phenylacetaldehyde, benzaldehyde was generated as a product from L-phenylalanine.

### (Test Example 7)

The amounts of the products phenylacetaldehyde and benzaldehyde generated were confirmed.

1 ml of 250 mM phosphate buffer (pH 6.0), 2.5 ml of 20 mM L-phenylalanine, 1 ml of 1 mass% apple polyphenol solution, and 1 ml of 0.5 mass% laccase solution were added to a 15 ml centrifuge tube, and thereafter, the reaction was carried out by shaking at 50°C for 1 hour. After completion of the reaction, the enzyme was inactivated by a boiling treatment for 10 minutes.

**[Table 14]**

| Reaction composition | Additive amount (ml) | Final concentration |
|---|---|---|
| 250 mM Phosphate buffer (pH 6.0) | 1 | 50 mM |
| 1 mass% Apple polyphenol solution | 0.5 | 0% or 0.1 mass% |
| 20 mM L-Phenylalanine | 2.5 | 10 mM |
| 0.5 mass% Laccase solution | 1 | 0% or 0.1 mass% |

After 0.8 g of NaCl had been dissolved in 4 ml of the reaction solution, solid-phase extraction was carried out using SPME Arrow, and the generated products were then analyzed by GC-MS.

**[Table 15]**

| Amino acid | Enzyme | Apple polyphenol | Benzaldehyde (ppm) | Phenylacetaldehyde (ppm) |
|---|---|---|---|---|
| L-Phenylalanine | - | - | 0.01 | Undetected |
| | + | - | 0.02 | Undetected |
| | - | + | 0.03 | 1.12 |
| | + | + | 2.22 | 9.24 |

| | | | | |
|---|---|---|---|---|
| ("+" indicates addition, and "-" indicates non-addition (* water was added instead)). | | | | |

When L-phenylalanine was used as a substrate, the use of apple polyphenol + enzyme generated approximately 70 times more BA (benzaldehyde) and approximately 8 times more PAA (phenylacetaldehyde) than when the only apple polyphenol was used. It is anticipated that benzaldehyde would be generated as a by-product after phenylacetaldehyde was generated.

## Claims

1. A method for producing phenylacetaldehyde, including
allowing a mediator to act on phenylalanine or an analogue thereof, in which
the mediator is a compound containing a ring structure having at least one hydroxyl group.

2. The method for producing phenylacetaldehyde according to claim 1, in which the allowing the mediator to act on phenylalanine or an analogue thereof is carried out in the presence of multicopper oxidase.

3. The method for producing phenylacetaldehyde according to claim 1 or 2, in which the mediator is a compound containing a ring structure having at least one hydroxyl group and an alkoxy group, or a compound containing a ring structure having two or more hydroxyl groups.

4. The method for producing phenylacetaldehyde according to claim 3, in which the ring structure is a benzene ring or a dihydrofuran ring.

5. The method for producing phenylacetaldehyde according to claim 1 or 2, in which the mediator is at least one type selected from the group consisting of DL-catechin, tannic acid, chlorogenic acid, caffeic acid, curcumin, hesperidin, L-DOPA, pyrogallol, vanillin, eugenol, gallic acid monohydrate, ferulic acid, isoeugenol, α-glucosylrutin and rutin.

6. The method for producing phenylacetaldehyde according to claim 1 or 2, in which the mediator is a compound contained in at least one type selected from the group consisting of an apple extract, pectin, a green coffee bean extract and a tea extract.

7. The method for producing phenylacetaldehyde according to claim 1 or 2, in which the mediator is a polyphenol.

8. The method for producing phenylacetaldehyde according to claim 1 or 2, in which the mediator is at least one type selected from the group consisting of α-glucosylrutin, chlorogenic acid, caffeic acid, tannic acid and rutin.

9. The method for producing phenylacetaldehyde according to claim 2, in which the multicopper oxidase is at least one type selected from phenol oxidase and bilirubin oxidase.

10. The method for producing phenylacetaldehyde according to claim 9, in which the multicopper oxidase is laccase.

11. The method for producing phenylacetaldehyde according to claim 1 or 2, in which the reaction temperature in the allowing the mediator to act on phenylalanine or an analogue thereof is 60°C or lower.

12. A phenylacetaldehyde-generating enzyme agent containing a mediator and multicopper oxidase, in which
the mediator is a compound containing a ring structure having at least one hydroxyl group.
